Europäisches Patentamt

European Patent Office

Office européen des brevets

(11) Numéro de publication: **0 377 393**
**A1**

## (12) DEMANDE DE BREVET EUROPEEN

(21) Numéro de dépôt: 89480179.4

(51) Int. Cl.⁵: **A61K 7/26**

(22) Date de dépôt: 28.11.89

(30) Priorité: 12.12.88 FR 8816430

(43) Date de publication de la demande:
11.07.90 Bulletin 90/28

(84) Etats contractants désignés:
AT BE CH DE ES FR GB GR IT LI LU NL SE

(71) Demandeur: **Cherkaoui, Zhour**
**94 Boulevard Maurice Langlet**
**F-06340 La Trinité(FR)**

(72) Inventeur: **Cherkaoui, Zhour**
**94 Boulevard Maurice Langlet**
**F-06340 La Trinité(FR)**

(74) Mandataire: **Hautier, Jean-Louis**
**Cabinet Hautier Office Méditerranéen de**
**Brevets d'Invention et de Marques 24 rue**
**Masséna**
**F-06000 Nice(FR)**

(54) Composition à base de produits naturels pour entretenir la bouche et les dents en exerçant une action bienfaisante sur les gencives.

(57) L'invention a pour objet une composition à base de produits naturels.

La composition selon l'invention est du type contenant une substance astringente qui favorise le resserrement des tissus, une substance qui rafermit les tissus et fait briller les dents, une substance qui est également astringente et caustique pour détruire le tartre et les déchets se trouvant entre et/ou sur les dents; elle est caractérisée par le fait que la substance astringente qui favorise le resserrement des tissus et agit sur la mauvaise haleine est l'alun (sulfate double de potassium et d'aluminium hydraté) ; la substance qui fortifie les gencives et qui fait briller les dents est de la peau de racine de noyer ; la substance qui est astringente et caustique est de la peau de grenade.

Composition dont l'application principale est la stomatologie.

## Composition à base de produits naturels pour entretenir la bouche et les dents en exerçant une action bienfaisante sur les gencives

L'invention a pour objet une composition à base de produits naturels pour entretenir la bouche et les dents en exerçant une action bienfaisante sur les gencives.

Ladite composition peut permettre d'obtenir une solution pour les bains de bouche ou une pâte pour dentifrice.

L'état de la technique peut être défini par les brevets suivants :

- FR-A-2.516.384 : l'invention concerne un nouveau dentifrice contenant des enveloppes de riz broyées à une dimension particulaire telle qu'elles traversent un tamis à mailles de soixante quatorze micromètres d'ouverture, de préférence de quarante quatre micromètres comme abrasif essentiel, en des proportions inférieures à 40% en poids de la composition totale.

- FR-A-2.522.500 : la présente invention concerne de nouveaux médicaments et cosmétiques caractérisés en ce qu'ils contiennent de la pulpe de potiron de Chine ou de cucurbitacées analogues.

- FR-A-2.505.654 : l'invention concerne une composition aqueuse comprenant une composition aromatisée à deux notes constituée d'huile essentielle et d'une oléorésine insoluble dans l'eau extraite de fruits secs, cette oléorésine produisant une sensation plus forte que l'huile essentielle et étant soluble dans cette huile essentielle, la composition aromatisante constituant 0,01 à 5% du poids de la composition aqueuse, l'oléorésine constituant environ 0,001 à 0,01% du poids de la composition aqueuse et le rapport pondéral de l'huile essentielle à l'oléorésine étant d'au moins environ 10/1.

- FR-A-2.488.796 : l'invention a pour objet une composition dentifrice utilisant un agent gélifiant essentiellement constitué par du xanthane et de la gomme de guar en des proportions particulières.

- EP A2 0051.752 : la présente invention a pour objet un dentifrice, sa méthode de préparation où le dentifrice contient de l'acide tannique, efficace contre la carie des dents, la pyorrhée et la gingivite, et en même temps est efficace pour prévenir le fait que les dents noircissent. Ce dentifrice est obtenu par des méthodes d'addition de substances au dentifrice, qui a été traité par réduction, en ajoutant du cuivre chlorophylline-sodium audit acide et en les mélangeant ensemble.

- EP A2-0117.905 : l'invention décrit un agent pour soins de la bouche, en particulier sous forme de dentifrice et de bain de bouche. Il présente une teneur en huile de millepertuis. L'utilisation de l'huile de millepertuis dans des agents pour soins de la bouche sert pour la prophylaxie de caries et de paradontoses par formation d'un film.

- EP A2-0 108.318 : la présente invention concerne une composition pour entretenir la bouche et les dents qui exerce une action bienfaisante sur les gencives et en particulier inhibe et réduit le saignement des gencives. Cette composition dentaire contient une combinaison de composés constitués d'urée et de marron d'inde.

- DE-A-2.637.862 : Produit pour l'hygiène dentaire caractérisé par le fait qu'en tant que chlorure de sodium il contient du sel de cuisine, du sel gemme ou du sel marin finement pulvérisé et qu'en tant que matière active du genre que l'on trouve dans les écorces de noyers, il contient de l'écorce de noyers finement pulvérisée.

- FR-A-616.204 : l'invention a pour objet le produit industriel nouveau constitué par une crème, ou une pâte destinée au massage des gencives, ledit produit présentant les caractéristiques suivantes :

la pâte est formée de soufre finement divisé et d'un astringent tel que l'alun, d'un hydrate de carbone tel que l'amidon, avec de l'eau, d'un véhicule liquide tel que la glycérine, et d'une substance, telle qu'une huile aromatique, possédant une saveur agréable.

- FR-A-1.478.371 : Procédé permettant de bronzer la peau pour une longue durée. Ce produit est composé d'extraits de plantes naturelles, il permet de faire bronzer la peau sans recourir au soleil et à l'air ni aux rayons X. Ce produit étant inoffensif se compose pour un litre d'eau :

Henné : 200g ;
Brou de noix : 10g ;
Extrait de grenade : 3g ;
Noir de fumée : 3g ;

L'été, ils peuvent se mélanger à l'huile d'olive vierge, pressée à froid.

- W. KERN et al. : "Hagers handbuch der pharmazeutishen praxis", édition 4, partie A : N-Q, 1977, pages 979-981, Springer-Verlag, Berlin, DE : Ce document dans son paragraphe 15 décrit les différentes applications de la grenade. Celle-ci est connue pour ses propriétés astringentes (gargarisme). Les caractéristiques telles que décrites dans l'invention ne sont pas mentionnées ni suggérées par ce document.

Ces différentes compositions ont toutes des actions spécifiques sur les dents et/ou sur les gencives.

La composition selon l'invention est à base de produits naturels compatibles notamment avec un traitement homéopathique. Cette composition permet de raffermir les gencives, d'éviter les saignements desdites gencives et le déchaussement des dents. Elle évite également la formation du tartre, l'éclatement de l'émail, les caries et la mauvaise haleine.

La composition selon l'invention est du type contenant une substance astringente qui favorise le resserrement des tissus, une substance qui rafermit les tissus et fait briller les dents, une substance qui est également astringente et caustique pour détruire le tartre et les déchets se trouvant entre et/ou sur les dents.

La substance astringente qui favorise le resserrement des tissus et agit sur la mauvaise haleine est l'alun (sulfate double de potassium et d'aluminium hydraté).

La composition est caractérisée par le fait qu'elle utilise une combinaison avec l'alun.

- La substance qui fortifie les gencives et qui fait briller les dents est de la peau de racine de noyer.

- La substance qui est astringente et caustique est de la peau de grenade.

Le procédé de fabrication de la composition, selon l'invention, réside dans les phases suivantes :

à partir de la quantité de produits suivants :

A- 200 gr. d'alun

B- 150 gr. de peau de racine de noyer

C- 100 gr. de peau de grenade - Les produits B et C (peau de racine de noyer et peau de grenade) sont séchés.

- La peau de racine de noyer est trempée dans deux litres d'eau fraiche, pendant environ une heure.

- Les produits A et C sont ensuite ·ajoutés dans l'eau de trempage qui contient le produit B. L'ensemble est bouilli.

- L'ensemble A + B + C est malaxé avec l'eau de cuisson.

- Puis l'ensemble est passé dans une passoire qui retient les produits trop volumineux et ce, pour les éliminer de la composition.

- L'ensemble A + B + C et l'eau de cuisson est mis à décanter dans un bocal étanche au réfrigérateur et ce, pendant environ quarante huit heures.

- Par filtrage, on sépare le liquide ainsi obtenu d'une crème plus lourde qui reste au fond du bocal.

- Ledit procédé permet d'obtenir environ 350 ml de liquide, 150 g de crème.

Le liquide ainsi obtenu est utilisé pour les bains de bouche, la crème peut être utilisée comme dentifrice.

est également astringente et caustique pour détruire le tartre et les déchets se trouvant entre et/ou sur les dents; la substance astringente qui favorise le resserrement des tissus et agit sur la mauvaise haleine est l'alun (sulfate double de potassium et d'aluminium hydraté) caractérisé par le fait que la substance qui fortifie les gencives et qui fait briller les dents est de la peau de racine de noyer ; la substance qui est astringente et caustique est de la peau de grenade.

2. Composition selon la revendication 1 caractérisée par le fait que la proportion des produits A, B, C est, au début du procédé de fabrication, la suivante :

A- 200 gr. d'alun

B- 150 gr. de peau de racine de noyer

C- 100 gr. de peau de racine de grenade

pour obtenir 350 ml de composition liquide pour les bains de bouche et 150 gr. de crème pour le dentifrice.

3. Procédé pour la fabrication de la composition selon l'une quelconque des revendications 1 ou 2 caractérisé par le fait qu'il réside dans les phases suivantes :

à partir de la quantité de produits suivants :

A- 200 gr. d'alun

B- 150 gr. de peau de racine de noyer

C- 100 gr. de peau de racine de grenade

- Les produits B et C (peau de racine de noyer et peau de racine de grenade) sont séchés.

- La peau de racine de noyer est trempée dans deux litres d'eau fraiche, pendant environ une heure.

- Les produits A et C sont ensuite ajoutés dans l'eau de trempage qui contient le produit B. L'ensemble est bouilli.

- L'ensemble A + B + C est malaxé avec l'eau de cuisson.

- Puis l'ensemble est passé dans une passoire qui retient les produits trop volumineux et ce, pour les éliminer de la composition.

- L'ensemble A + B + C et l'eau de cuisson est mis à décanter dans un bocal étanche au réfrigérateur et ce, pendant environ quarante huit heures.

- Par filtrage, on sépare le liquide ainsi obtenu d'une crème plus lourde qui reste au fond du bocal.

- Ledit procédé permet d'obtenir environ 350 ml de liquide, 150 g de crème.

**Revendications**

1. Composition selon l'invention du type contenant une substance astringente qui favorise le resserrement des tissus, une substance qui rafermit les tissus et fait briller les dents, une substance qui

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | DE-A-2 637 862 (D.S. MALLA) <br> * Revendications * <br> --- | 1 | A 61 K 7/26 |
| D,A | W. KERN et al.: "Hagers Handbuch der pharmazeutischen Praxis", edition 4, partie A: N-Q, 1977, pages 979-981, Springer-Verlag, Berlin, DE <br> * Page 979: "Punica" - page 981, sous "Anwendung" * <br> --- | 1 | |
| D,A | FR-A- 616 204 (C.M. GEARHART) <br> * Résumé * <br> --- | 1 | |
| D,A | FR-A-1 478 371 (ABIDELLAH) <br> * Résumé; colonne de gauche, paragraphes 7,8 * <br> ----- | 1 | |

DOMAINES TECHNIQUES RECHERCHES (Int. Cl.5)

A 61 K

Le présent rapport a été établi pour toutes les revendications

| Lieu de la recherche | Date d'achèvement de la recherche | Examinateur |
|---|---|---|
| LA HAYE | 24-04-1990 | WILLEKENS G.E.J. |